# EUROPEAN PATENT SPECIFICATION

(11) **EP 2 226 634 B1**
(45) Date of publication and mention of the grant of the patent: **21.10.2015**
(21) Application number: 10155606.6
(22) Date of filing: 05.03.2010
(51) Int. Cl.: B82Y 15/00, B82Y 25/00, G01N 33/543

(54) **Fluorescent colloidal nanocapsules, a process for their production and use in cells selection assays**
Kolloidale Fluoreszenz-Nanokapseln, Verfahren zu deren Herstellung und Verwendung in Zellenauswahlassays
Nanocapsules colloïdales fluorescentes, leur procédé de production et leur utilisation dans des analyses de sélection de cellules

(30) Priority: 06.03.2009 IT TO20090169
(43) Date of publication of application: 08.09.2010
(73) Proprietor: Fondazione Istituto Italiano Di Tecnologia, 16163 Genova (IT)
(72) Inventor: Pellegrino, Teresa, 73100 Lecce (IT); Di Corato, Riccardo, 73100 Lecce (IT); Piacenza, Philomena, 44809 Bochum (DE); Musaro' , Mariarosaria, 73032 Andrano(Lecce) (IT); Manna, Liberato, 16145 Genova (IT); Cingolani, Roberto, 73100 Artesano (Lecce) (IT)
(74) Representative: Rambelli, Paolo

(56) References cited:
- US-A1- 2006 057 211
- YANG ET AL: "Fluorescent magnetic nanohybrids as multimodal imaging agents for human epithelial cancer detection" BIOMATERIALS, ELSEVIER SCIENCE PUBLISHERS BV., BARKING, GB, vol. 29, no. 16, 10 March 2008 (2008-03-10), pages 2548-2555, XP022559052 ISSN: 0142-9612
- LU J ET AL: "Manganese ferrite nanoparticle micellar nanocomposites as MRI contrast agent for liver imaging" BIOMATERIALS, ELSEVIER SCIENCE PUBLISHERS BV., BARKING, GB, vol. 30, no. 15, 20 February 2009 (2009-02-20), pages 2919-2928, XP026010878 ISSN: 0142-9612
- YANG X ET AL: "Folate-encoded and Fe3O4-loaded polymeric micelles for dual targeting of cancer cells" POLYMER, ELSEVIER SCIENCE PUBLISHERS B.V, GB, vol. 49, no. 16, 28 July 2008 (2008-07-28) , pages 3477-3485, XP022940245 ISSN: 0032-3861 [retrieved on 2008-06-11]
- LEE C M ET AL: "SPION-loaded chitosan-linoleic acid nanoparticles to target hepatocytes" INTERNATIONAL JOURNAL OF PHARMACEUTICS, ELSEVIER BV, NL, vol. 371, no. 1-2, 24 December 2008 (2008-12-24), pages 163-169, XP026071376 ISSN: 0378-5173
- LARSEN B A ET AL: "Controlled aggregation of superparamagnetic iron oxide nanoparticles for the development of molecular magnetic resonance imaging probes" 2 July 2008 (2008-07-02), NANOTECHNOLOGY 20080702 INSTITUTE OF PHYSICS PUBLISHING; DIRAC HOUSE GB, VOL. 19, NR. 26 , XP002555187 * the whole document *
- JIAN YANG ET AL: "Quantum Dot Nanobarcodes: Epitaxial Assembly of Nanoparticle-Polymer Complexes in Homogeneous Solution", JOURNAL OF THE AMERICAN CHEMICAL SOCIETY, vol. 130, no. 15, 1 April 2008 (2008-04-01), pages 5286-5292, XP055146886, ISSN: 0002-7863, DOI: 10.1021/ja710934v

## Description

The present invention relates to a method of preparation of magnetic fluorescent nanobeads suitable to simultaneous performing of bioseparation and biodetection.

In particular, the invention relates to a method which allows to obtain nanobeads comprising a core made of an aggregate of a plurality of superparamagnetic nanoparticles and a shell comprising an amphiphilic polymer to which surface fluorophore molecules are bound.

The combination of magnetic and fluorescent properties in a single nanostructured entity allows its use in several biomedical applications, as biodetection, sorting and bioseparation, as well as a therapeutic tool for drug delivery.

A nano-system designed for bio-separation must be able to accumulate promptly to a magnet, while its size must be small enough to ensure a high sensitivity.

Moreover, said nanosystem must be easily dispersible in the medium where it has to interact with cells or markers.

Recently several works have described the use of amphiphilic polymers to enwrap hydrophobic colloidal magnetic nanoparticles (such as quantum dots, gold and iron oxide nanoparticles) in order to obtain a dispersion of hydrophobic particles in water solutions. The enwrapping by amphiphilic polymer allows the transfer of nanoparticles from an organic solvent to a water solution and the chemical modification and bioconjugation of the particles, since biological molecules can develop covalent bonds to the polymer surface.

Pellegrino et al. in "Nano Letters", 2004. vol. 4, n. 4, 703-707 described a general processing strategy for transfer of hydrophobic nanocristals to a water solution using an amphiphilic polymer of poly(maleic anhydride-alt-1-tetradecene). In this method, the polymer is added to a nanocrystal solution in chloroform, then the solvent is evaporated; then a triamine solution in chloroform is added as a cross-linking agent, again followed by solvent evaporation and addition of a borate water buffer solution, followed by sonication.

This process leads to cross-linking of polymeric chains around single nanoparticles, so that the resulting nanoparticles, which are individually polymer coated, lack magnetic properties, even if this method is applicable to iron oxide magnetic nanoparticles.

Di Corato et al. in "J. Mater. Chem.", 2008, 18, 1191-1196, describe a variation of the above mentioned process which uses poly (maleic anhydride -alt-1-ottadecene).

Cheng-An J. Lin et al. in "Small", 2008, 4, n. 3, 334-341, describe the synthesis of an amphiphilic polymer for coating and functionalisation of nanoparticles . The incorporation of fluoresceine-ammine in the amphiphilic polymer is described as an example of functionalisation with an organic fluorophore. However the authors observe strong fluorescence quenching in case of polymeric shell containing fluorescein and including gold particles.

Jian Yang et al. in "J. Am. Chem. Soc.", 2008, 130, 5286-5292, describe a method for the preparation of nanobeads including quantum dots, based on the epitaxial growth of nanoparticles-amphiphilic polymer complexes in a homogeneous solution, utilising poly (maleic anhydride-octadecene) (PMAO). Also in this method the quantum dots are precoated by PMAO by means of hydrophobic interaction and quantum dots-polymer complexes form nanobeads following a solvent polarity increase; nanobeads are further stabilized by polymeric chains crosslinking using diamine.

The article reports that more than 250 quantum dots can be included in a nanobead having a diameter of about 100 nm. However, in this case too fluorescence quenching has been observed.

Since the fluorophores are very close to each other, fluorescence intensity does not increase linearly with the increase of fluorophores concentration and it may even decrease.

Yang et al., "Fluorescent magnetic nanohybrids as multimodal imaging agent for human epithelial cancer detection", BIOMATERIALS, ELSEVIER SCIENCE PUBLISHERS BV., BARKING, GB, vol. 29, No. 16, 10 March 2008, pages 2548-2555 describes Cetuximab conjugated fluorescent magnetic nanohybrids synthesized for detection of human epithelial cancer via magnetic resonance and optical imaging. The magnetic nanohybrids are prepared by a method wherein a fluorescent amphiphilic copolymer is put in water and magnetic nanocristals are put in chloroform; fluorescent magnetic nanoparticles are obtained by a nanoemulsion method wherein the two solvents, water and chloroform do not form a single phase.

Larsen B A et al., "Controlled aggregation of superparamegnetic iron oxide nanoparticles for the development of molecular magnetic resonance imaging probes", 2 July 2008, NANOTECHNOLOGY INSTITUTE OF PHYSICS PUBLISHING; DIRAC HOUSE GB, vol. 19, No. 26, describes a method for synthesizing superparamagnetic iron oxide multi-nanoparticles aggregates as molecular magnetic resonance imaging contrast agent. The approach utilizes organic acids/base interaction in the colloid to induce highly controllable nanoparticle aggregation.

One aim of this invention is to provide a method to prepare fluorescent nanobeads free from the quenching phenomenon.

Another aim of the invention is to provide a method leading to the preparation of nanobeads characterized by strong magnetic properties and therefore able to provide a faster response to a separating magnet in comparison to single particles, even if administrated in small quantity, (diluted, i.e in solution and aggregates forms).

Another aim of the invention is to provide a method for the preparation of nanobeads which allows to achieve the above mentioned results and to reduce the number and the complexity of the required synthesis phases for preparing the nanobeads.

These aims are reached by means of a method having the characteristics described in the attached claims.

Nanobeads described herein which can be obtained using the method according to the invention comprise superparamagnetic nanoparticles, enwrapped by an amphiphilic polymer to which surface fluorophore molecules are bound.

The method according the invention comprises the following processing phases.
1) Preparation of colloidal nanoparticles made of ferromagnetic materials having nanometer crystallite sizes so that said nanoparticles show superparamagnetic behavior at room temperature (or anyway at the typical temperatures of use of said nanobeads, e.g. from 5°C to 60°C).

Usually iron oxide, manganese-iron and mixed cobalt-iron oxides (e.g. MnFe₂O₄, CoFe₂O₄) or transition metals alloys (e.g. CoPt₃, FePt) colloidal nanoparticles are used.

The preparation step is carried out preferably by thermal decomposition of a organometallic precursor (for example iron pentacarbonyl), which is injected into an organic solution of surfactant agents or an organic solvent comprising surfactants; the reaction mixture is then heated to the precursor decomposition temperature. The reaction mixture is then kept at this temperature for the time required to the growth of the crystallites and then cooled. The nanocrystals are then precipitated, preferably in ethanol.

The organic solvent, when used, is preferably an apolar or moderately polar solvent and the surfactant or mixture of surfactants is preferably selected among alkyl-carboxylic acid, alkylamine, alkyl-phosphonic acid, alkyl-thiols and alkyl-diols and their mixtures.

The method yields nanoparticles having a surfactant coating on their surface, making said nanoparticles soluble in organic solvents as toluene, chloroform and tetrahydrofuran.

Typically, nanoparticles so manufactured have sizes ranging from 5 nm to 40 nm, depending on their material composition.

### 2) Preparation of nanobeads

The processing steps to prepare the nanobeads comprise:
i) adding a specific quantity of a solution of amphiphylic polymer functionalised with a fluorophore in an apolar solvent to a solution of surfactant-coated nanoparticles in an apolar solvent, where said solvents are mixable to obtain a monophasic mixture of said nanoparticles and polymer and
ii) later adding a solvent more polar than the solvent or solvents utilised in i) and mixable to this solvent/s and wherein said nanoparticles are insoluble and the used polymer is partially soluble.

The amphiphilic polymers which can be used within the invention exhibit a main hydrophilic poly maleic anhydride backbone having hydrophobic alkyl side chains; typically, said polymers are obtained by conjugation of alkyl side chains having amine ends to a poly-anhydride chain. Typically the side hydrophobic chains can have from 4 to 20 carbon atoms, in particular from 6 to 20 atoms, side chains having from 14 to 18 carbon atoms are preferred; in particular within the invention poly(maleic anhydride-alt-1-octadecene) (P_{C18}) or poly(maleic anhydride-alt-1-tetradecene) (P_{C14}) are used. Free anhydride groups of these polymers in the main chain can be modified with functional ligands having terminal amino group, as for example fluorophore molecules.

In step i), the amphiphilic polymer is dissolved in an apolar solvent, tetrahydrofuran preferably, and preferably the same solvent is used for the magnetic nanoparticles colloidal solution.

In step ii), an aprotic polar solvent is preferably used, as acetonitrile (ACN) o dimethyl sulfoxide (DMSO), even if polar protic solvents as methanol or ethanol can be used. The solvent volume can be established on the basis of a Teas diagram, according the particular polymer used. Solubility properties are meant at room temperature.

According to experiments carried out by the inventors, the parameters leading to the formation of nanobeads and controlling their sizes and the radial distribution of superparamagnetic nanoparticles inside said nanobeads are essentially the polymer solubility in the final solvents mixture (THF/ACN or THF/H₂O etc.) and the polymer concentration.

In the particular case of P_{C18}, the addition of ACN, DMSO or methanol leads to nanobeads formation in a wide range of experimental conditions. When adding ACN, the nanobeads are characterized by a core of aggregated nanoparticles, coated by an outer polymeric shell. Higher concentrations of polymer in the starting solution lead to a thicker polymer shell; however, a polymer shell is formed even at the lowest concentration of polymer used (for example 1 mM). The polymer concentration in the solvent can be changed over a wide range and is preferably comprised between ImM and 5 mM for a 0,2 ml solution.

The total nanobead size (core of aggregated particles plus polymer shell) can be varied between 100 and 400 nm and the size of the aggregated particles core can be varied between 100 and 170 nm.

In general, however, the thicker the polymer shell the more stable the resulting beads are in water.

On the contrary, adding a solvent wherein the polymer is completely insoluble (for example water) leads to small-size nanobeads (about 30nm), each of them including a very small number of superparamagnetic nanoparticles. On the other hand, adding a solvent wherein the polymer is completely soluble (for example acetone), leads to nanobeads including a higher number of nanoparticles; however it is impossible to appropriately control the nanobeads size distribution.

A possible mechanism for the nanobeads formation is the following: the change in polarity of the solution upon addition of the solvent induces a conformational reorganization of the polymer in solution and simultaneously promotes hydrophobic interaction among surfactant-coated particles as well as between the alkyl chains of amphiphilic polymer and the superparamagnetic surfactant-coated nanoparticles. However said mechanism is not to be intended as a constraint within the scope of this invention.

Furthermore, the polymer provides the beads surface with carboxylic groups, deriving from the partial hydrolysis of the anhydrides present on the polymer chains. The negative charges introduced at the beads surface make them insoluble in polar solvents like ethanol or water.

As above mentioned, the method according to the invention contemplates the production of nanobeads wherein the amphiphilic polymer which enwraps the superparamagnetic aggregates is functionalized with fluorescent molecules, which makes the nanobeads suitable for both bioseparation and biodetection. To this aim it is preferable to perform the polymer functionalization first and subsequently to carry out the procedure as described above for the preparation of the beads.

It is preferable to use oligothiophene molecules as fluorophores, even if other organic fluorescent dyes can be used, such as fluorescein. The anhydride groups of the polymer chain, which are opened using fluorophores having amine ends, are exploited to bind the fluorophore to polymer. In particular, within the invention amino-oligothiophenes (NH₂-OTF) e N-hydroxysuccinimide-oligothiophenes (NHS-OTF) have been utilized. When NHS-OTF is used, a first reaction step between NHS-OTF and a diaminic ligand is required, followed by the attachment of the conjugated amino-ligand-OTF to the polymer . The previous polymer functionalization with the fluorophore does not influence the nanobeads preparation phase, but it allows the production of nanobeads wherein the fluorophore molecules are separated from the core by the polymeric shell, which acts as a barrier, avoiding fluorescence quenching.

Further features and advantages of the method according the invention will be clearly apparent from the following embodiments.

In the attached drawings:
- Fig. la is a nanobeads synthesis scheme using acetonitrile addition to a solution of P_{C18} and iron oxides nanoparticles in THF;
- Fig. 1b shows TEM images of single nanobeads of different diameters obtained by acetonitrile addition at increasing polymer concentrations (1, 2, 3 and 4 mM, respectively);
- Fig. 2 is a FT-IR spectrum of the nanobeads in water; the most significant features are the following: the peak at 1720 cm⁻¹ corresponding to the C = O stretching of the carboxylic groups originated from the hydrolysis of the polymer anhydride groups and the shoulder in the range 3400 - 2500 cm⁻¹ corresponding to the broad O-H stretching overlapping the peaks at 2920 and 2855 cm⁻¹ which are probably due to C-H stretching of the aliphatic polymer chain;

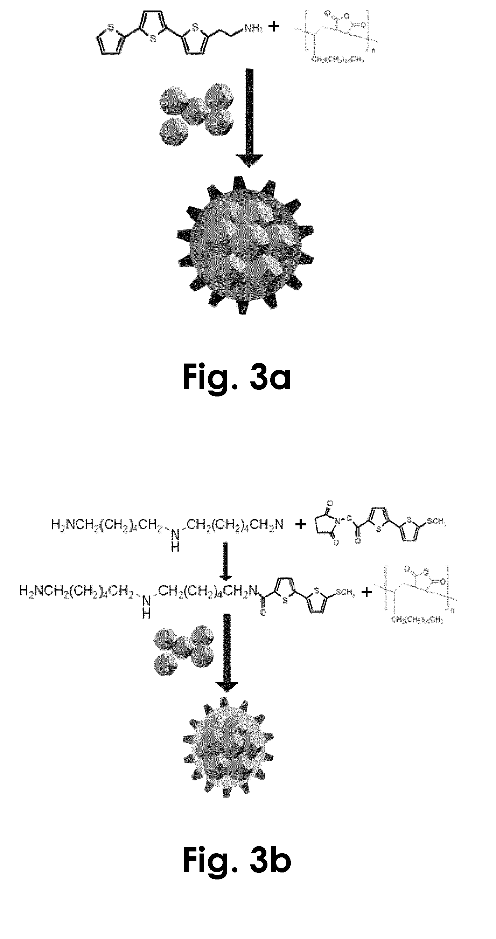

- Fig. 3a is an outline of the fluorescent nanobeads formation when using NH₂-OTFs;
- Fig. 3b is an outline of the fluorescent nanobeads formation when using NHS-OTFs;
- Fig. 3c shows PL spectra of fluorescent nanobeads functionalized with different amount of NH2-OTFs; an increased fluorescent signal is detected when increasing the NH₂-OTF1 molecules bound to the polymer ;
- Fig. 4a shows cell sorting tests results; KB cells doped with different concentrations of magnetic nanobeads (corresponding to 10, 16, 32,5 and 65 µg/ml of iron) have been accumulated to the magnet after 5, 10, 15 and 30 minutes; cell counts are plotted versus incubation time;
- Fig. 4b is a diagram showing cell viability tests on KB cells doped with increased amounts of magnetic nanobeads;
- Fig. 4c shows sections of KB cells doped with fluorescent magnetic nanobeads functionalized with OTF; and

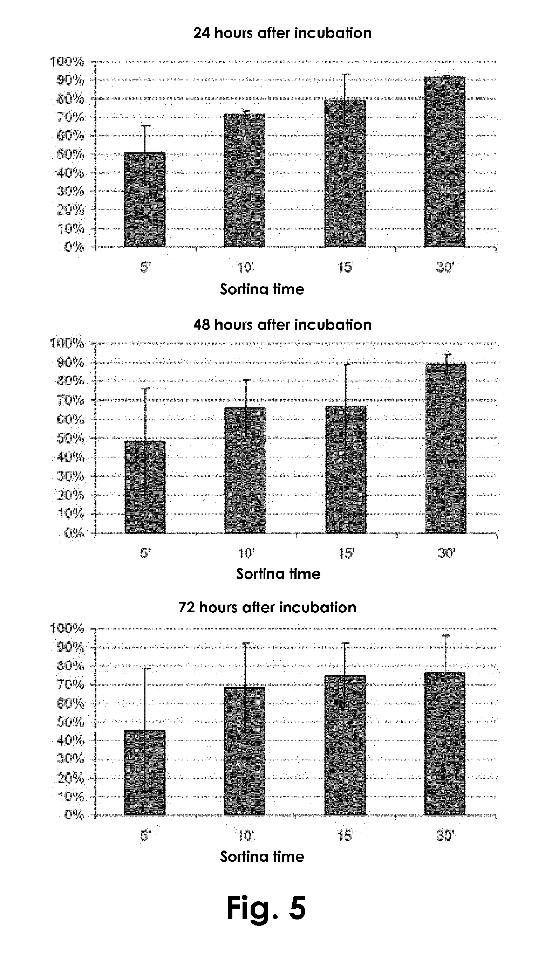

- Fig. 5 shows cell sorting tests at different times.

### Example 1 - synthesis of magnetic nanoparticles

Iron oxide spheres were synthesized by a modified protocol reported by Sun et al. in "Journal of the American Chemical Society", 2002, 124, (28), 8204-8205. 13 nm iron oxide spheres were obtained by a seeded-growth approach starting from 6-7 nm seeds. In a typical synthesis for iron oxide seeds, 1 mM of Fe(CO)₅ was injected into a three-necked flask containing 1.5 mM of oleic acid (OLAC), 0,74 mM of oleylamine (OLAM) and 1,25 mM of 1,2-esadecandiol in 10 ml of 1-octadecene (ODE).

The reaction mixture was heated up to 280°C and kept at this temperature for 1 hour. To obtain 13 nm spheres, two further injections were made to the above described reaction mixture. In detail, a precursor solution (consisting of 2 mM of Fe(CO)₅ and 3 mM of OLAC in 5 ml of ODE) was injected drop-wise at a rate of 0.1 ml/minute. The mixture was kept at 280°C for 1 hour after each injection. Finally, the mixture was allowed to cool down to 130°C. Then, the flask was exposed to air and kept at this temperature for 1 hour. After the synthesis, the sample was washed at least three times by precipitation with ethanol, after which the nanocrystals were re-suspended in THF.

### Example 2 - preparation of Beads

A nanoparticles solution is prepared dissolving 13 nm γ-Fe₂O₃ nanoparticles, which were previously prepared according to example 1, in THF in order to reach a concentration of 2 µM. A polymer solution is prepared dissolving P_{C18} in THF to reach a polymer concentration of 50 mM (in monomers units) and adding 20 µL of previously prepared nanoparticles solution to 8 µl of the polymer solution and then adding THF to reach a final volume of 200 µl. The mixture is shaken for 30 minutes on a vortex system at 1000 rpm (the use of magnetic stir bar is avoided, as the beads tend to stick to it).

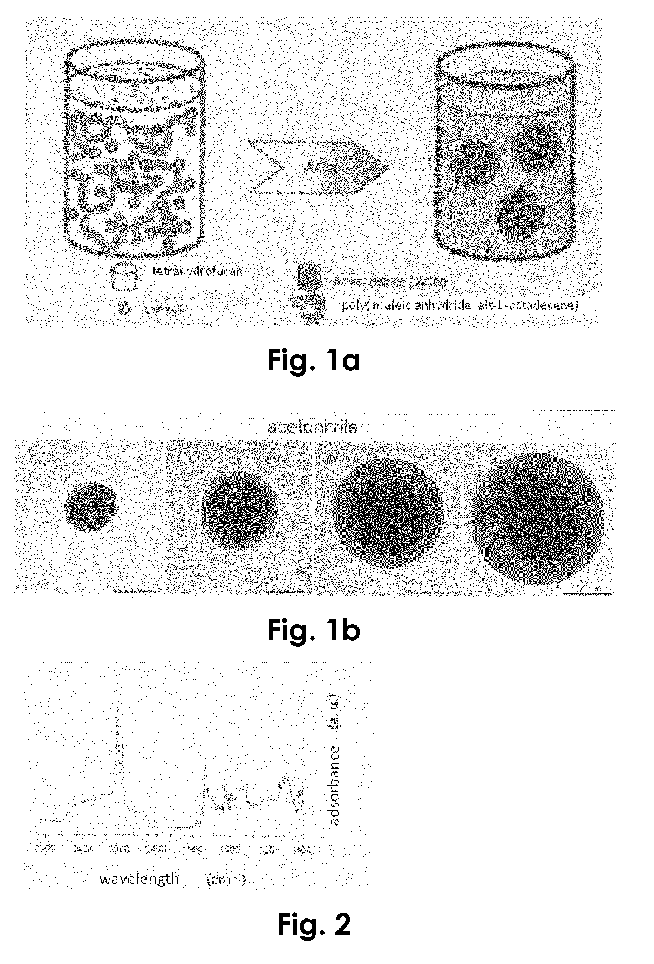

Subsequently, 800 µl of ACN are added at a rate of 0.25 ml/minute. After the beads are formed, a magnet (NdFeB, 0.5 T) is positioned close to one side of the vial, which lead to quantitative accumulation of the beads to that side of the vial. Afterwards, the mother solution in the vial is replaced by a borate phosphate buffer (50 mM, pH 9) and the beads are re-dissolved in the buffer upon removal of the magnet. The beads prepared by addition of ACN were characterized by a core of aggregated particles encased by a shell of polymer. The control of the thickness of the polymer shell in these beads (Fig.1b) depends on the amount of P_{C18} polymer solution (50 mM in THF) added to the solution of γ-Fe₂O₃ nanoparticles; said quantity could be 4, 8, 12 and 16 µl, respectively.

In general, critical parameters that control the beads formation are the shaking speed at which the polymer and the iron oxide nanoparticles are mixed, the mixing time before the addition of the solvent of different polarity and the rate at which such solvent is added.

### Example 3 - NH₂-OTFs and NHS-OTFs functionalization

To bind OTFs to the magnetic nanobeads two approaches were followed. In the first approach NH₂-OTFs are bound to the P_{C18} polymer. More specifically, in a series of experiments, different volumes of NH₂-OTF solution in THF (5 mM) (1, 10, 20, 50 and 100 µl, respectively) were mixed to 10 µl of the P_{C18} polymer solution in THF (50 mM) in order to reach well-defined rations of NH₂-OTF molecules added every 100 monomer units (1, 10, 20, 50, and 100, respectively) of the polymer. Then an amount of THF was added to reach a final volume of 200 µl and the solutions were shaken at room temperature overnight. The resulting mixtures were added to a powder of γ-Fe₂O₃ nanoparticles of 13 nm (obtained by drying 20 µl of a 2 µM solution of nanoparticles) and shaken at 1000 rpm for 30 minutes. Afterwards 800 µl of ACN were added at a rate of 0.25 ml / minute.

In the second approach, NHS-OTFs were grafted to the P_{C18} polymer. First, 20 µl of a NHS-OTF solution in THF (5 mM) were mixed with 20 µl of a solution of bis(hexamethylene)triamine in THF (5 mM). Then an amount of THF was added to reach a final volume of 190 µl and the solution was vortexed overnight. The resulting mixture was reacted with 10 µl of P_{C18} in THF (50 mM) and left under vortexing for additional 12 hours at room temperature. The reaction mixture was finally added to a dried powder of γ-Fe₂O₃ nanoparticles of 13 nm (obtained by drying 20 µl of a 2 µM solution of nanoparticles) and to this solution 800 µl of ACN were added at a rate of 0.25 ml/minute.

For the purification of the fluorescent and magnetic nanobeads (MFNBs hereinafter) from the excess of the OTF derivates, the nanobeads solution is added to a 10% sucrose solution and centrifuged at 3000 g for 2 hours on 12 mL tubes. The OTF derivates are floating on the upper part of the sucrose solution, while the beads are recovered from the bottom of the tube by applying a magnet to the vial wall. The sucrose solution is replaced by a borate buffer solution 50 mM (pH 9).

### Example 4 - cell culture

The human epidermoid carcinoma cells, known as KB cells (ATCC # CCL-17) are grown continuously as a monolayer at 37°C and under 5% CO2 atmosphere in RPMI- 1640 medium, supplemented with L-glutamine (2 ml), penicillin (100 units/ml), streptomycin (100 µg/ml), and 10% heat inactivated fetal bovine serum (FBS).

### Example 5- cell sorting assay

The KB cells are first seeded into a 6-well plate (200000 cells/well), after which a solution containing the magnetic nano-beads was added to them and the dish was incubated at 37°C for 24 h. The nanobeads were added to the cells at different concentrations (corresponding to total concentrations of iron equal to 6.5, 16, 32.5 and 65 µg/ml, respectively). After incubation, the medium was removed and the adhering cells were washed twice with phosphate buffer.

Soon after, the cells were trypsinized and 1 ml of fresh medium was added into each well. The medium containing the cells was collected from each well and kept on ice during the time needed for the cell count, as the cells were not fixed. The total number of cells per well was counted. For the cell sorting experiments 1 ml of the solution containing a known number of cells was placed in a glass vial. Four glass vials containing the same number of cells were exposed simultaneously to the magnet for four different times intervals (5, 10, 15, and 30 minutes, respectively).

After each vial has been exposed to the magnet, the portion of cells in suspension (not attracted to the magnet) and the portion of cells accumulated to the magnet were collected separately and in both portions the number of cells were counted. The cell sorting experiment was repeated for each sample at least on four independent samples.

### Example 6 - cytotoxicity assay

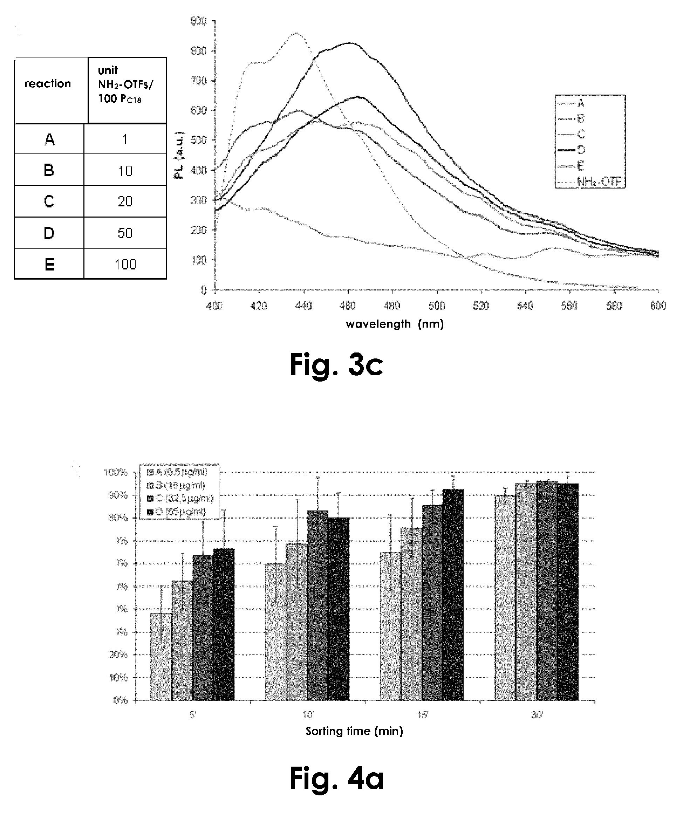

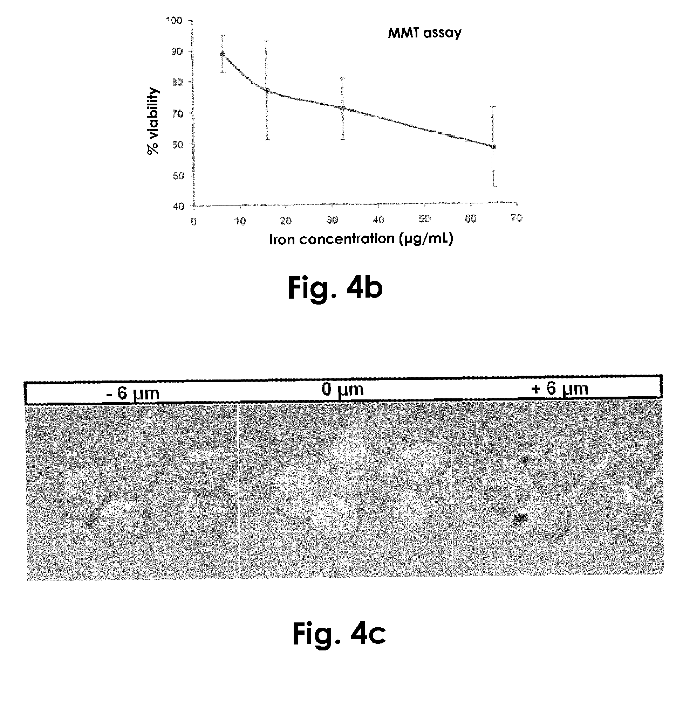

In order to estimate the toxicity of the conjugates, a viability test was carried out using the 3-(4,5-dimethyl-2-thiazolyl)-2,5-diphenyl-2H-tetrazolium bromide assay (MTT assay). The assay results are reported in the diagram of Fig. 4b.

The MTT test allowed us to asses that the cytotoxicity of the MFNBs on the cells was less than 20% for iron concentrations equal to 16 µg/ml, while it reached 40% for the highest iron concentration administered (65 µg/ml), suggesting that on the isolated cells there is no need of detach the MFNBs since non significant cell toxicity is observed.

The method according the invention allows to obtain the following main advantages:
- possibility of tuning the internal structure size of the nanobeads.
- production of nanobeads in a wide size range (between 30 and 400 nm), compatible with the bio-system (cells or markers) and able to respond promptly to a magnet for bio-separation tasks;
- the process doesn't involve separated, sequential steps of clustering of magnetic nanoparticles to form a nanostructure and fluorescent coating of the nanostructure;
- high photostability is achieved as the polymer shell eliminates the risk of fluorescence quenching by the magnetic core;
- low toxicity nanobeads and tunable fluorescent signal from OTFs are achieved;
- the method further allows to functionalize the nanobeads surface with a variety of molecules to detect specific bio-targets;
- the nanobeads have high stability and solubility in the biological medium.

## Claims

1. A method for the preparation of magnetic fluorescent nanobeads, comprising an amphiphilic polymer shell and a core having superparamagnetic behaviour including an aggregate of ferromagnetic or ferrimagnetic nanoparticles, **characterised in that** it comprises the steps of:
a) providing colloidal nanoparticles, coated with a surfactant, said nanoparticles having such size as to show superparamagnetic properties at room-temperature;
b) adding a solution of amphiphylic polymer functionalized with a fluorophore in an apolar solvent to a solution of said particles in an apolar solvent to obtain a monophasic mixture of said superparamagnetic nanoparticles and polymer;
c) adding to the mixture obtained in b) a solvent more polar than the solvent/s used in b) and mixable with said solvent/solvents used in b), thereby forming a single phase, in which more polar solvent said nanoparticles are insoluble and the used polymer is only partially soluble.

2. The method according to claim 1, **characterised by** the fact that the amphiphilic polymer is an alkyl poly (maleic anhydride) polymer having C₄-C₂₀ alkyl side chains.

3. The method according to claims 1 or 2, wherein the amphiphilic polymer is selected between poly (maleic anhydride-octadecene) and poly (maleic anhydride-tetradecene).

4. The method according to any of the previous claims, wherein in step b) said nanoparticles and said polymer are dissolved in an apolar solvent, comprising tetrahydrofuran.

5. The method according to any of the previous claims, wherein the solvent in step c) is selected among acetonitrile, dimethyl sulfoxide, methanol and ethanol.

6. The method according to any of the previous claims, wherein in step b) the amphiphilic polymer is present in an amount between 1 and 5mM per 0.2 ml of the mixture obtained in b).

7. The method according to any of the previous claims, wherein, in step b), iron oxide (Fe₂O₃), mixed iron oxides, particularly MnFe₂O₄, CoFe₂O₄, or transition metal alloys, particularly CoPt₃, FePt, colloidal nanoparticles are used.

8. The method according to any of the previous claims, wherein said nanoparticles exhibiting superparamagnetic behaviour have a size ranging between 5 nm and 40 nm.

9. The method according to any of the previous claims, wherein the amphiphilic polymer used in b) is bound to oligothiophene, hydroxysuccinimide oligothiophene molecules or fluorescein.

10. The method according to any of claims 1 to 9, wherein said nanobeads are separated from the solvent by the application of a magnetic field.

## Patentansprüche

1. Verfahren zur Herstellung von magnetischen fluoreszierenden Nanokügelchen, umfassend eine amphiphile Polymerschale und einen Kern mit superparamagnetischem Verhalten einschließlich einem Zuschlag von ferromagnetischen oder ferrimagnetischen Nanopartikeln,
**dadurch gekennzeichnet,**
**dass** es die folgenden Schritte umfasst:
a) Bereitstellung von mit einem oberflächenaktiven Mittel beschichteten kolloidalen Nanopartikeln, die Nanopartikel sind von solcher Größe, dass sie superparamagnetische Eigenschaften bei Raumtemperatur aufweisen;
b) Hinzufügen einer Lösung aus einem amphiphilen Polymer, funktionalisiert mit einem Fluorophor in einem apolaren Lösungsmittel zu einer Lösung der Teilchen in einem apolaren Lösungsmittel, um eine einphasige Mischung aus den superparamagnetischen Nanopartikeln und Polymeren zu erhalten;
c) Hinzufügen eines Lösungsmittels zu der in b) erhaltenen Mischung, wobei das Lösungsmittel eine höhere Polarität aufweist als das/die in b) verwendete(n) Lösungsmittel sowie mischbar mit dem/den in b) verwendeten Lösungsmittel(n) ist, wodurch eine einzige Phase gebildet wird, wobei in der höherpolaren Lösung die Nanoteilchen unlöslich sind und das verwendete Polymer nur teilweise löslich ist.

2. Verfahren nach Anspruch 1,
**dadurch gekennzeichnet,**
**dass** es sich bei dem amphiphilen Polymer um ein Alkyl-Poly (Maleinsäureanhydrid) Polymer mit C₄-C₂₀-Alkyl-Seitenketten handelt.

3. Verfahren nach einem der Ansprüche 1 oder 2,
**dadurch gekennzeichnet,**
**dass** bei dem amphiphilen Polymer zwischen Poly (Maleinsäureanhydrid-Octadecen) und Poly (Maleinsäureanhydrid-Tetradecen) gewählt wird.

4. Verfahren nach einem der vorhergehenden Ansprüche,
**dadurch gekennzeichnet,**
**dass** in Schritt b) die Nanopartikel und das Polymer in einem apolaren Lösungsmittel, welches Tetrahydrofuran enthält, gelöst werden.

5. Verfahren nach einem der vorhergehenden Ansprüche,
**dadurch gekennzeichnet,**
**dass** das Lösungsmittel in Schritt c) aus Acetonitril, Dimethylsulfoxid, Methanol und Ethanol ausgewählt wird.

6. Verfahren nach einem der vorhergehenden Ansprüche,
**dadurch gekennzeichnet,**
**dass** in Schritt b) das amphiphile Polymer in einer Menge zwischen 1 und 5 mM je 0,2 ml der nach b) erhaltenen Mischung vorliegt.

7. Verfahren nach einem der vorhergehenden Ansprüche,
**dadurch gekennzeichnet,**
**dass** in Schritt b), Eisenoxid (Fe₂O₃), Eisenmischoxide, insbesondere MnFe₂O₄, CoFe₂O₄ oder Übergangsmetall-Legierungen, insbesondere CoPt₃, FePt, als kolloidale Nanopartikel verwendet werden.

8. Verfahren nach einem der vorhergehenden Ansprüche,
**dadurch gekennzeichnet,**
**dass** die superparamagnetisches Verhalten aufzeigenden Nanopartikel eine Größe im Bereich zwischen 5 nm und 40 nm haben.

9. Verfahren nach einem der vorhergehenden Ansprüche,
**dadurch gekennzeichnet,**
**dass** das in b) verwendete amphiphile Polymer, an Oligothiophen, hydroxysuccinimide oligothiophene Moleküle oder Fluorescein gebunden wird.

10. Verfahren nach einem der Ansprüche 1 bis 9,
**dadurch gekennzeichnet,**
**dass** die Nanokügelchen durch Anwendung eines Magnetfeldes aus dem Lösungsmittel getrennt werden.

## Revendications

1. Méthode de préparation de nanobilles fluorescentes magnétiques, comprenant une enveloppe en polymère amphiphile et un noyau présentant un comportement superparamagnétique comprenant un agrégat de nanoparticules ferromagnétiques ou ferrimagnétiques, **caractérisée en ce qu'**elle comprend les étapes :
a. de fourniture de nanoparticules colloïdales, enduites d'un surfactant, lesdites nanoparticules ayant une taille leur permettant de posséder des propriétés superparamagnétiques à température ambiante,
b. d'ajout d'une solution de polymère amphiphile fonctionnalisée avec un fluorophore dans un solvant apolaire dans une solution desdites particules dans un solvant apolaire afin d'obtenir un mélange monophasique desdites nanoparticules superparamagnétiques et dudit polymère,
c. d'ajout au mélange obtenu en b) d'un solvant plus polaire que le(s) solvant(s) utilisé(s) en b) et miscible avec ledit(lesdits) solvant(s) utilisé(s) en b), formant ainsi une phase simple ; dans ledit(lesdits) solvant(s) plus polaire(s), lesdites nanoparticules sont insolubles et le polymère utilisé n'est que partiellement soluble.

2. Méthode selon la revendication 1, **caractérisée en ce que** le polymère amphiphile est un alkyl poly (anhydride maléique) polymère possédant des chaînes latérales alkyle C₄-C₂₀.

3. Méthode selon les revendications 1 ou 2, dans laquelle le polymère amphiphile choisi est soit le poly (anhydride maléique-octadécène) soit le poly (anhydride maléique-tétradécène).

4. Méthode selon une des revendications précédentes, dans laquelle, dans l'étape b), lesdites nanoparticules et ledit polymère sont dissous dans un solvant apolaire, comprenant du tétrahydrofurane.

5. Méthode selon une des revendications précédentes, dans laquelle le solvant de l'étape c) est choisi entre l'acétonitrile, le diméthylsulfoxyde, le méthanol et l'éthanol.

6. Méthode selon une des revendications précédentes, dans laquelle, dans l'étape b), le polymère amphiphile est présent dans une quantité comprise entre 1 et 5 mM pour 0,2 ml de mélange obtenu en b).

7. Méthode selon une des revendications précédentes, dans laquelle, dans l'étape b), on utilise de l'oxyde de fer (Fe₂0₃), des oxydes de fer mélangés, en particulier du MnFe₂O₄, du CoFe₂0₄, ou des alliages de métaux de transition, en particulier du CoPt₃, du FePt, des nanoparticules colloïdales.

8. Méthode selon une des revendications précédentes, dans laquelle lesdites nanoparticules présentant un comportement superparamagnétique ont une taille comprise entre 5 nm et 40 nm.

9. Méthode selon une des revendications précédentes, dans laquelle le polymère amphiphile utilisé en b) est lié à des molécules d'oligothiophène, d'hydroxysuccinimide oligothiophène ou de fluorescéine.

10. Méthode selon une des revendications 1 à 9, dans laquelle lesdites nanobilles sont séparées du solvant par l'application d'un champ magnétique.
